# EUROPEAN PATENT APPLICATION

(11) **EP 4 711 409 A1**
(43) Date of publication of application: **18.03.2026**
(21) Application number: 24199915.0
(22) Date of filing: 12.09.2024
(51) Int. Cl.: C08J 11/16, B01J 35/39, C08J 11/14, C08J 11/24, C07C 211/00, C07C 209/14, C07C 31/18, C07C 29/128, B01J 12/00, B01J 23/04, B01J 23/00

(54) **DEGRADATION OF POLYURETHANE MATERIALS TO THEIR MONOMERS**

(71) Applicant: DePoly SA, 1950 Sion (CH)
(72) Inventor: Anderson, Samantha, 1009 Pully (CH); Uran, Pelin, 1009 Pully (CH); Bila, Hale, 1030 Bussigny (CH); Soodi, Sanaz, 1950 Sion (CH)
(74) Representative: Prins Intellectual Property AG

(57) **Abstract**

A process for depolymerization of polyurethane plastic waste into monomers of diamine and polyol comprising the steps of: a. providing in a reactor a reaction mixture comprising: i. polyurethane plastic waste, ii. a solvent, iii. a base in an amount to obtain a pH of 8 to 14 in the reaction mixture, and iv. a UV light active metal oxide catalyst; b. stirring the reaction mixture under UV light for a predetermined time, during which C-N and/or C-Q bonds of the urethane groups of the polyurethane plastic waste is cleaved to obtain diamines and polyols; c. recovering the diamines and polyols from the reaction mixture; wherein the steps a. and b. are performed at a temperature of 20°C to approx. 60°C and ambient pressure.

## Description

### Technical Field

The invention relates to a process for degradation of polyurethane waste material to their monomers, in particular diamines and polyols, under mild conditions at temperatures below 80°C and at ambient pressure.

### Technical Background

Polyurethane (PU) is a class of polymers composed of organic units joined by carbamate (urethane) links. PU is a widely used polymer for different applications in different forms, such as elastomers, adhesives, paints or foams. PU is the 6th most used polymer on the world, corresponding to approx. 8% of the total plastic production. There are different methods for recycling PU waste, including mechanical, biological, and chemical treatment technologies. Mechanically recycled PU materials cannot be turned into new plastic products easily, mainly due to the physical characteristics of it. Biological methods such as using enzymes can transform PU waste into valuable products; however, the degradation time is long (days to weeks) and it is a high cost process. Chemical recycling methods are promising, yet the known techniques require harsh conditions. Known chemical recycling methods include acidolysis, glycolysis and hydrolysis. Generally, high temperatures are used to convert polyurethane chains back into raw material. Hydrolysis is a promising chemical method, and the urethane linkage reacts with water to produce diamine and polyol, which are the main components for the new PU material synthesis. However, hydrolysis of PU commonly requires 150-200 °C in pressurized liquid water or by steam at 200-450 °C. An example of such a method is described in EP0990674.

US5208379 for example suggests a method to hydrolyse a polyurethane produced by reacting an active hydrogen-containing polyether and an organic polyisocyanate, which comprises contacting said polyurethane with water in the presence of an effective amount of a strong base and an activating agent selected from the group consisting of quaternary ammonium salts containing at least 15 carbon atoms and organic sulfonates containing at least 7 carbon atoms. Even though US5208379 discloses in the general description that the reaction temperature may be chosen in a range of from 80 to 225 °C, example 19 shows that at 120 °C only partial hydrolysis took place and example 18 shows that at 140°C yields were only 70%.

WO2023078802 describes a method of solvolyzing a polyurethane. Solvolysis may be carried out as hydrolysis. The polyurethane is dispersed at an average particle size of 0.1 to 12 mm and the polyurethane content in the dispersion is in a range of 4 to 20 percent by weight. Dispersing the polyurethane is carried out in a temperature range between ambient temperature and 120°C and at ambient pressure. The hydrolysis however is then carried out at a temperature from 80°C to 200°C for up to 14 hours.

There still exists a need for a more effective, inexpensive, robust and practical technology for degrading plastic polyurethane waste, and simultaneously producing diamines and polyols that form the polyurethane, where the entire reaction may be performed at lower temperatures and ambient pressure.

### Summary of the Invention

It is an objective of the invention to provide an energy efficient process for degrading at least some types of PU. It is a further objective of the invention to provide a process which uses low molecular alcohols and/or water as only solvents, and to avoid other solvents, which may be harmful to the environment. It is a further objective of the invention that the process can be efficiently performed without pretreatment of the polyurethane waste material, except for crushing or chopping.

At least one of the objectives of the present invention is achieved by a process for depolymerization of polyurethane plastic waste according to claim 1. The process depolymerizes polyurethane plastic waste into monomers of diamine and polyol and comprises the steps of: (a.) providing in a reactor a reaction mixture comprising: (i.) polyurethane plastic waste, (ii.) a solvent, (iii.) a base in an amount to obtain a pH of 8 to 14 in the reaction mixture, and (iv.) a UV light active metal oxide catalyst; (b.) stirring the reaction mixture under UV light for a predetermined time, during which C-N and/or C-O bonds of the urethane groups of the polyurethane plastic waste is cleaved to obtain diamines and polyols; and (c.) recovering the diamines and polyols from the reaction mixture. The steps a. and b. may be performed at a temperature of 20 to approx. 60°C and ambient pressure.

Surprisingly, the inventors found out that the process can efficiently cleave urethane groups of polyurethane waste without any pretreatment except for chopping the material into smaller pieces and at low temperatures and ambient pressure. Energy consumption is thereby considerably decreased compared to the known methods. It is e.g. not required to bring the polyurethane waste in suspension before adding it to the reaction mixture. Also, no other ingredients were necessary, such that the reaction mixture may consist of (i.) polyurethane plastic waste, (ii.) a solvent, (iii.) a base in an amount to obtain a pH of 8 to 14, preferably 13 to 14, in the reaction mixture, and (iv.) a UV light active metal oxide catalyst.

The method worked particularly well when the polyurethane plastic waste is of polyurethane produced by reacting an aromatic isocyanate with a polyol, where the isocyanate is methylene diphenyl diisocyanate (MDI), preferably 4,4'-mehtylenedianiline, or toluene diisocyanate (TDI), preferably 2,4-toluenediamine. Such polyurethanes have the following formula: 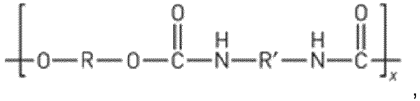 where R derives from the polyol and R' derives from the isocyanate.

The polyols may differ in molecular weight and number of hydroxyl groups. More flexible PU is made of higher molecular weight polyols, typically with a molecular weight from 2,000 to 10,000. More rigid PU is made from lower molecular weight polyols.

The recovered diamine monomers from these polyurethanes were mehtylenedianiline (MDA) (e.g. 4,4'-mehtylenedianiline) or toluenediamine (TDA) (e.g. 2,4-toluenediamine). Depending on the type of polyurethane waste, the recovered polyols differed and were the monomers of which the polyurethane were composed.

Further embodiments of the invention are set forth in the dependent claims.

In some embodiments the only solvent of the reaction mixture may be an alcohol or a water-alcohol mixture, wherein the alcohol is selected from the group consisting of methanol, ethanol, propanol, butanol, pentanol or combinations thereof. A volumetric water-alcohol ratio of the water-alcohol mixture may be in the range of 0.1 to 4, preferably approx. 0.25 (20:80).

In some embodiments the amount of polyurethane plastic waste in the reaction mixture may be about 5 to 20 % by weight. The polyurethane plastic waste may be pretreated by cutting, crushing, grinding into flakes or other fragments or pulverizing. Cutting, crushing, grinding into flakes or other fragments or pulverizing may be the only pretreatment used. No pre-washing or chemical pretreatment is necessary. It is possible to directly subject collected polyurethane plastic waste without pre-sorting to the process. In other words, the polyurethane plastic waste may include contaminations such as non-plastic material but also other types of plastic material.

In some embodiments the base may be an alkali metal hydroxide or an alkaline earth metal hydroxide, preferably NaOH or KOH.

In some embodiments the UV light active metal oxide catalyst may have a band gap in the range of 3.0 to 3.6 eV, preferably 3.0 to 3.4 eV, more preferably 3.0 to 3.2 eV. The UV light active metal oxide catalyst may be selected from the group of TiO2, V2O5, Cr2O3, CrO3, Mn2O3, FeO, Fe2O3, Fe3O4, Co2O3, NiO, CuO, Cu2O, ZnO, ZrO2, Nb2O5, Mo2O3, RuO, RuO2, RuO4, RhO2, Rh2O3, PdO, Ag2O, Ag2O2, CdO, In2O3, Al2O3, La2O3, CeO2, Ce2O3, HfO2, Ta2O5, WO3, ReO2, ReO3, Re2O3, OsO2, OsO4, lrO2, PtO2, Au2O3, Li2O, Na2O, K2O, MgO, CaO, SrO, BaO, and combinations thereof. A preferred metal oxide catalyst is TiO2 or P25 or ZnO.

The metal oxide catalyst or the combination of metal oxide catalysts may be bound to a metal selected from the group comprising Pt, Rh, Pd, Ag, Au, Zn, Ni, and Ir. Thus, the metal oxide catalyst or the combinations of the metal oxides catalyst chemically or physically mixed from the group disclosed above may be bound on their surface to a metal selected from the group comprising Pt, Rh, Pd, Ag, Au, Zn, Ni, and Ir.

In some embodiments the predetermined time for stirring the reaction mixture may be 30 min to 360 min, preferably 3 to 6 hours. The process may be a batch process or a continuous process.

In some embodiments the ratio by weight of polyurethane:base may be 1:1 to 1:3, preferably about 1:3.

In some embodiments the ratio by weight of polyurethane:metal oxide may be 1:0.005 to 1:0.03, preferably about 1:0.01.

In some embodiments the UV light may have a wavelength in the range of 100 to 400 nm, preferably 315 to 400 nm; and an intensity in the range of 50 to 150 mW/cm2, preferably around 100 mW/cm2.

In some embodiments the recovery of diamines and polyols may include the steps of: (a.) concentration of reaction mixture to remove the reaction solvents, (b.) isolation of polyol and diamine with acid-base extraction.

The polyurethane waste material may be obtained from post-consumer goods, such as beverage bottles, non-beverage containers, food containers, packaging materials, carpeting, clothing, textile fibers, plastic tubes, plastic films, plastic sheets, wrapping materials, foam material, and synthetic fibers. In some embodiments, the polyurethane is not pretreated (no pretreatment and/or no cleaning is required; the polyurethane as-is is put in contact with the metal oxide in a solution, such as an aqueous alcoholic solution, in the presence of a base and stirred under UV light). In some embodiments, the polyurethane can undergo cutting, crushing, grinding or pulverizing into flakes or other fragments. The size of the fragments may be in the range of 1 mm to 30 mm.

### Embodiments of the Invention

The sample which was used for the experiments was a shredded membrane of polyurethane (PU) and the reactions were performed at 50-60 °C with TiO2 as a photocatalyst and UV light with a wavelength in the range of 315 to 400 nanometers as a source of photon. The depolymerization of PU yields two types of monomers, polyols and diamines. The polyols and diamines can be recovered, and the yield referred to in the following is the yield according to the unreacted portion of PU after depolymerization.

Different conditions were tried to show proof of concept of the PU depolymerization.

### pH range:

The PU depolymerization was performed at different pH (= 8, 9, 10, and 14) in a pH range from 8 to 14 and the highest yield was obtained at pH=14. The other experimental conditions were:
- polyurethane : NaOH ratio at 1:3 (by weight)
- H2O:ethanol ratio at 20:80 (volumetric)
- temperature adjusted at 50-60 °C,
- TiO2 as metal oxide catalyst, and
- reaction time set at 4 hr.

The results at different pH are shown in below Table 1.

**Table 1**

| **pH** | **Yield (%)** |
|---|---|
| 8 | 12 |
| 9 | 15 |
| 10 | 23 |
| 14 | 76 |

### H2O: ethanol ratio:

The PU depolymerization was performed at the different ranges of water to ethanol ratio and the highest value was obtained at a ratio H2O:ethanol of 20:80. The other experimental conditions were:
- polyurethane : NaOH ratio at 1:3 (by weight)
- pH at 14
- temperature adjusted at 50-60 °C,
- TiO2 as metal oxide catalyst, and
- reaction time set at 4 hr.

The results at different H2O: ethanol ratio (volumetric) are shown in below Table 2.

**Table 2**

| **H2O: ethanol ratio (volumetric)** | **Yield (%)** |
|---|---|
| 80:20 | 0 |
| 50:50 | 66 |
| 20:80 | 76 |
| 10:90 | 62.3 |

### MOH base:

The PU depolymerization was performed with two different MOH bases, namely NaOH and KOH. The other experimental conditions were:
- polyurethane : MOH ratio at 1:3 (by weight)
- pH at 14
- H2O:ethanol ratio at 20:80 (volumetric)
- temperature adjusted at 50-60 °C,
- TiO2 as metal oxide catalyst, and
- reaction time set at 4 hr.

The results are shown in below Table 3.

**Table 3**

| **MOH base** | **Yield (%)** |
|---|---|
| NaOH | 76 |
| KOH | 51 |

### Reaction time:

The PU depolymerization was performed at different reaction times in the range of 30 to 360 minutes. The other experimental conditions were:
- polyurethane : NaOH ratio at 1:3 (by weight)
- pH at 14
- H2O:ethanol ratio at 20:80 (volumetric)
- temperature adjusted at 50-60 °C
- TiO2 as metal oxide catalyst

The results are shown in below Table 4.

**Table 4**

| **reaction time (minutes)** | **Yield (%)** |
|---|---|
| 30 | 36 |
| 60 | 43 |
| 120 | 48 |
| 240 | 76 |
| 360 | 78 |

### PU: NaOH ratio:

The PU depolymerization was performed at different ratio of polyurethane : NaOH of 1:1, 1:2, and 1:3 (by weight). The best performance was with a ratio of 1:3. The other experimental conditions were:
- pH at 14
- H2O:ethanol ratio at 20:80 (volumetric)
- temperature adjusted at 50-60 °C
- TiO2 as metal oxide catalyst

The results are shown in below Table 5.

**Table 5**

| **PU** : **NaOH ration** (by weight) | **Yield (%)** |
|---|---|
| 1:1 | 57 |
| 1:2 | 50 |
| 1:3 | 76 |

### ZnO as metal oxide catalyst

The PU depolymerization was performed with ZnO instead of TiO2 as catalyst. The experimental conditions were:
- polyurethane : NaOH ratio at 1:3 (by weight)
- pH at 14
- H2O:ethanol ratio at 20:80 (volumetric)
- temperature adjusted at 50-60 °C
- reaction time set at 4 hr

The yield compared to the unreacted polyurethane was 70 %.

### Type of PU material used:

The PU depolymerization was performed with different polyurethane samples. The other experimental conditions were:
- polyurethane : NaOH ratio at 1:3 (by weight)
- pH at 14
- H2O:ethanol ratio at 20:80 (volumetric)
- temperature adjusted at 50-60 °C
- TiO2 as metal oxide catalyst
- reaction time set at 4 hr

The results are shown in below Table 6.

**Table 6**

| **PU sample** | **Yield (%)** |
|---|---|
| Membrane 1: shredded | 76 |
| Membrane 2: shredded | 95 |
| Flexible foam 1 | 60 |
| Flexible foam 2 | 100 |
| Flexible foam 3 | 79 |
| Flexible foam 4 | 89 |

Membrane 1 and membrane 2 were of polyurethane made of MDI as aromatic isocyanate and different polyols. The flexible foams 1 to 4 were of polyurethane made of TDI as aromatic isocyanate and different polyols. The polyols differed in molecular weight and number of hydroxyl groups. More flexible PU is typically made of higher molecular weight polyols, typically with a molecular weight from 2,000 to 10,000. More rigid PU is typically made from lower molecular weight polyols.

## Claims

1. Process for depolymerization of polyurethane plastic waste into monomers of diamine and polyol comprising the steps of:
a. providing in a reactor a reaction mixture comprising:
i. polyurethane plastic waste,
ii. a solvent,
iii. a base in an amount to obtain a pH of 8 to 14, preferably 13 to 14, in the reaction mixture, and
iv. a UV light active metal oxide catalyst;
b. stirring the reaction mixture under UV light for a predetermined time, during which C-N and C-O bonds of the urethane groups of the polyurethane plastic waste are cleaved to obtain diamines and polyols;
c. recovering the diamines and polyols from the reaction mixture;
wherein the steps a. and b. are performed at a temperature of 20°C to approx. 60°C and ambient pressure.

2. Process according to claim 1, wherein the polyurethane plastic waste is of polyurethane produced by reacting an isocyanate with a polyol, having the formula: 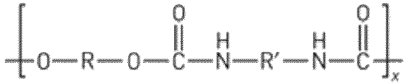 where R derives from the polyol and R' derives from the isocyanate, preferably methylene diphenyl diisocyanate (MDI) or toluene diisocyanate (TDI).

3. Process according to claim 1 or 2, wherein the only solvent of the reaction mixture is an alcohol or a water-alcohol mixture, wherein the alcohol is selected from the group consisting of methanol, ethanol, propanol, butanol, pentanol or combinations thereof.

4. Process according to claim 3, wherein a volumetric water-alcohol ratio of the water-alcohol mixture is in the range of 0.1 to 4.

5. Process according to one of the preceding claims, wherein the amount of polyurethane plastic waste in the reaction mixture is about 5 to 20 % by weight.

6. Process according to one of the preceding claims, wherein the base is an alkali metal hydroxide or an alkaline earth metal hydroxide, preferably NaOH or KOH.

7. Process according to one of the preceding claims, wherein the UV light active metal oxide catalyst has a band gap in the range of 3.0 to 3.6 eV, preferably 3.0 to 3.4 eV, more preferably 3.0 to 3.2 eV.

8. Process according to one of the preceding claims, wherein the UV light active metal oxide catalyst is TiO2, P25 or ZnO.

9. Process according to one of the preceding claims, wherein the metal oxide catalyst or the combination of metal oxide catalysts is bound to a metal selected from the group comprising Pt, Rh, Pd, Ag, Au, Zn, Ni, and Ir.

10. Process according to one of the preceding claims, wherein the predetermined time is 30 min to 360 min, preferably 3 to 6 hours.

11. Process according to one of the preceding claims, wherein the ratio by weight of polyurethane:base is 1:1 to 1:3, preferably about 1:3.

12. Process according to one of the preceding claims, wherein the ratio by weight of polyurethane:metal oxide is 1:0.005 to 1:0.03, preferably about 1:0.01.

13. Process according to one of the preceding claims, wherein the UV light has a wavelength in the range of 100 to 400 nm, preferably 315 to 400 nm; and an intensity in the range of 50 to 150 mW/cm2, preferably around 100 mW/cm2.

14. Process according to one of the preceding claims, wherein the recovery of diamines and polyols includes the steps of:
a. concentration of reaction mixture to remove the reaction solvents,
b. isolation of polyol and diamine with acid-base extraction.

15. Process according to one of the preceding claims, wherein the polyurethane plastic waste is pretreated by cutting, crushing, grinding into flakes or other fragments or pulverizing.
